# EUROPEAN PATENT SPECIFICATION

(11) **EP 3 490 587 B1**
(45) Date of publication and mention of the grant of the patent: **16.06.2021**
(21) Application number: 17754521.7
(22) Date of filing: 26.07.2017
(51) Int. Cl.: A61K 38/46, A61P 31/18

(54) **PROPHYLACTIC PROTECTION AGAINST VIRAL INFECTIONS**
PROPHYLAKTISCHER SCHUTZ VOR VIRUSINFEKTIONEN
PROTECTION PROPHYLACTIQUE CONTRE LES INFECTIONS VIRALES

(30) Priority: 26.07.2016 US 201662367050 P; 28.04.2017 US 201715582133
(43) Date of publication of application: 05.06.2019
(73) Proprietor: Squiquera, Luis, 1425 Buenos Aires (AR); Hodge, Thomas, Athens, GA 30606 (US); Sulley, Jamie, La Jolla, California 92037 (US)
(72) Inventor: Squiquera, Luis, 1425 Buenos Aires (AR); Hodge, Thomas, Athens, GA 30606 (US); Sulley, Jamie, La Jolla, California 92037 (US)
(74) Representative: Pearl Cohen Zedek Latzer Baratz UK LLP
(86) International application number: PCT/US2017/043984
(87) International publication number: WO 2018/022774

(56) References cited:
- WO-A1-2016/028634
- US-A1- 2004 072 910
- US-A1- 2012 121 569
- US-A1- 2013 022 589
- S. K. SAXENA ET AL: "Inhibition of HIV-1 Production and Selective Degradation of Viral RNA by an Amphibian Ribonuclease", JOURNAL OF BIOLOGICAL CHEMISTRY, vol. 271, no. 34, 23 August 1996 (1996-08-23), pages 20783-20788, XP055202278, ISSN: 0021-9258, DOI: 10.1074/jbc.271.34.20783
- YOULE R J ET AL: "RNase inhibition of human immunodeficiency virus infection of H9 cells", PROCEEDINGS OF THE NATIONAL ACADEMY OF SCIENCES, NATIONAL ACADEMY OF SCIENCES, vol. 91, no. 13, 1 June 1994 (1994-06-01), pages 6012-6016, XP002082703, ISSN: 0027-8424, DOI: 10.1073/PNAS.91.13.6012

## Description

### FIELD OF THE DISCLOSURE

The present disclosure relates to prophylactic treatment of viral infections using compositions comprising ribonucleases. More particularly, the disclosure relates to prophylactic treatment of sexually-transmitted viral infections, such as HIV and HPV infections, using topical ranpirnase compositions.

### BACKGROUND

Sexually transmitted infections, and particularly HIV, pose a significant public health threat. At present, individuals wishing to protect themselves against such infections rely upon mechanical measures (such as condoms and dental dams) to prevent them from coming into contact with their partner's bodily fluids, which may contain HIV. These measures are not optimal because some individuals are reluctant to use them. Recently, the use of orally administered antiretrovirals (e.g. tenofovir) has been proposed as pre-exposure prophylactic treatment. While oral prophylaxis is effective, it suffers from significant disadvantages. Oral prophylaxis must be used consistently for a prolonged period and its effectiveness is reduced or even eliminated if the patient is not fully compliant. Other oral medications can adversely affect the efficacy of oral prophylaxis. Furthermore, oral prophylactic medications are associated with side effects such as nausea and/or diarrhea.

Accordingly, there is a need for the development of prophylactic measures that are easy to use and not associated with side effects such as nausea and/or diarrhea.

### SUMMARY OF THE DISCLOSURE

The present application provides a composition comprising a ribonuclease and, optionally, one or more pharmaceutically acceptable excipients for use in prophylactically treating a subject from contracting a sexually-transmitted viral infection, wherein said composition is applied topically, and wherein the ribonuclease is a member of the ribonuclease A superfamily.

In one embodiment, the ribonuclease is selected from the group consisting of: ranpirnase, the '805 variant, Amphinase 2, and rAmphinase 2.

In some embodiments, the composition for prophylactic treatment comprises a personal lubricant

In some embodiments, the composition for prophylactic treatment is a gel, cream, ointment, lotion, solution, suspension, or a spray.

In one embodiment, the composition for prophylactic treatment comprises an effective amount of the ribonuclease and glycerin, hydroxy ethylcellulose, chlorhexidine gluconate, gluconolactone, methylparaben, and sodium hydroxide (e.g., KY -Jelly®).

In one embodiment, the composition for prophylactic treatment comprises an effective amount of the ribonuclease and glycerin, propylene glycol, sorbitol, hydroxyethylcellulose, benzoic acid, methylparaben, and sodium hydroxide.

In certain embodiments, the ribonuclease is present in the composition in an amount from about 0.01% by weight to about 10% by weight, based on the total weight of the composition. In certain other embodiments, the ribonuclease is present in the composition in an amount from about 0.1% by weight to about 1% by weight, based on the total weight of the composition. In some embodiments, the ribonuclease is present in the composition in an amount of about 1%, about 5%, or about 10%, by weight, based on the total weight of the composition.

In certain embodiments, the composition is applied prior to or during sexual intercourse. In particular aspects, the composition may be applied one to five times a day.

The composition may be applied topically to body regions that are exposed to sexually transmitted viruses.

In certain embodiments, the compositions of the present disclosure are for use in protecting subjects from contracting sexually-transmitted viruses such as herpes simplex viruses (HSV), human papillomaviruses (HPV), human immunodeficiency virus (HIV), hepatitis B and C virus, and cytomegalovirus.

In certain embodiments, the ranpirnase is present in the composition at a concentration of about 5 µg/ml to about 100 µg/ml or about 6 µg/ml to about 66 µg/ml.

### BRIEF DESCRIPTION OF THE DRAWINGS

**Figure 1** is a graph showing the cumulative HIV infection of individual rectal tissue biopsies at 14 days as a function of ranpirnase concentration using an explant challenge model.

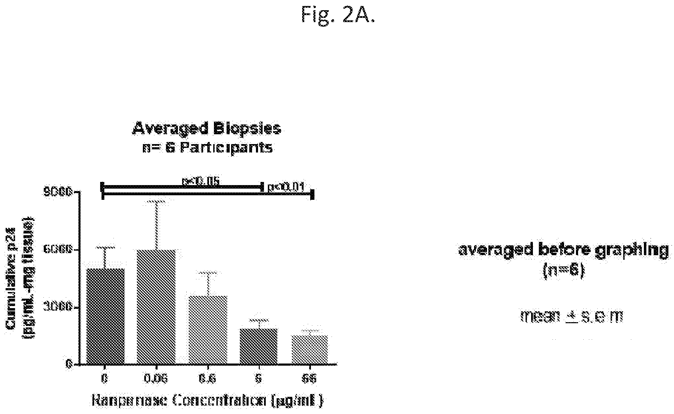

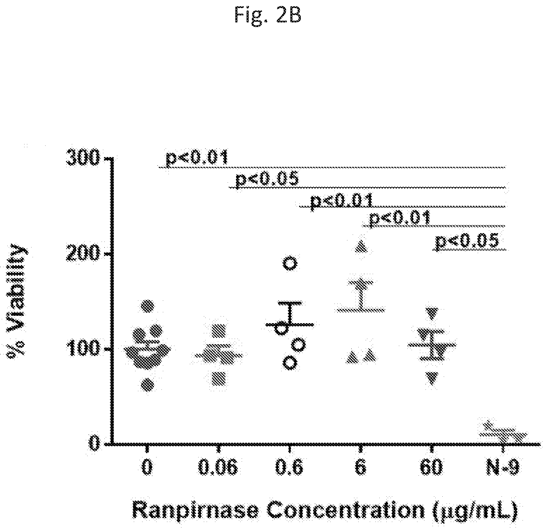

**Figure 2A** is a graph showing the cumulative averaged HIV infection of 5 rectal tissue biopsy triplets as a function of ranpirnase concentration. Specifically, dose-dependent reduction in Day 14 cumulative p24 release from HIV-1Bal infected biopsies without toxicity by ranpirnase. Figure 2A. Explants were co-exposed to 1x10⁵ virons/mL HIV-1 Bal and drug for 2 hours. Supernatant was collected for a 14 day culture period and assayed for p24 levels using the AlphaLISA Kit, (N=6 individuals with 3 biopsies each). Statistical significance was determined using standard mixed effect model with p<0.05. **Figure 2B** shows explant viability analysis. Explants were incubated for 2 hours in the presence of drug prior to viability analysis by MTT. Data are presented as percent viability relative to control biopsies not exposed to drug. N-9 is positive control, included to ensure proper assay function. Data are presented as mean +/- s.e.m. (N=3-9). Statistical significance was determined using standard mixed effect model with p<0.05.
**Figure 3** is a graph displaying the data in Figure 2 in a different format to clearly show the standard error of the mean at each ranpirnase concentration.
**Figure 4** shows a vaginal ring such as would be used in one of the embodiments of the invention.

### DETAILED DESCRIPTION

The present disclosure is based, in part, on the discovery that pre-treatment of a subject using compositions comprising a ribonuclease can protect the subject from acquiring sexually-transmitted viral infections.

Accordingly, the present application provides a composition comprising a ribonuclease and, optionally, one or more pharmaceutically acceptable excipients, for use in prophylactically treating a subject from contracting a sexually-transmitted viral infection, wherein said composition is applied topically, and wherein the ribonuclease is a member of the ribonuclease A superfamily. The subject is a mammal, in particular, human.

In one embodiment, the ribonuclease composition can be in the form of a gel, liquid, cream, ointment, lotion, solution, suspension, or a spray. These pharmaceutical forms have been defined by US Pharmacopeia (www.usp.org/sites/default/files/usp pdf/EN/USPNF/transdermalStimArticle.pdf) which describes therein formulations of gels, liquids, creams, ointments, lotions, solutions, and sprays. Further descriptions on the development of generic transdermal product have been described by Chang et al. (Chang, R.-K., Raw, A., Lionberger, R., & Yu, L. (2013). Generic Development of Topical Dermatologic Products: Formulation Development, Process Development, and Testing of Topical Dermatologic Products. The AAPS Journal, 15(1), 41-52. Examples on the proposed delivery systems for ranpirnase are described below.

In one embodiment, the ribonuclease is ranpirnase. Ranpirnase is an RNase isolated from oocytes of the leopard frog *Rana pipiens* which is disclosed in U.S. Pat No. 5,559,212, and is also known as Onconase®. The amino acid sequence of ranpirnase is provided in SEQ ID NO: 1. Ranpirnase has been tested and found to be cytotoxic to cancer cells because of its enzymatic activity against RNA.

A variant of ranpirnase is disclosed in U.S. Pat No. 5,728,805 (hereinafter, the "'805 variant"). The '805 variant is also an RNase, and has likewise been found to be cytotoxic to certain cancer cells. The '805 variant is a close variant of ranpirnase; its amino acid sequence is identical to that of ranpirnase except that it has valine instead of isoleucine at position 11, asparagine instead of aspartic acid at position 20, and arginine instead of serine at position 103 of the ranpirnase amino acid sequence. In some embodiments, the '805 variant is referred to as "Val 11, Asn20, Argl03-Ranpirnase". The amino acid sequence of the '805 variant is provided in SEQ ID NO: 2.

Amphinase 2 is also an RNase. It is the protein identified as 2325p4 in U.S. Pat. No. 6,239,257 and it also has been found to be cytotoxic to cancer cells. The amino acid sequence of Amphinase 2 is provided in SEQ ID NO: 3.

Recombinant Amphinase 2 ("rAmphinase 2") is similar to Amphinase 2, but has a Met residue at position -1 and lacks glycan moieties that are located in Amphinase 2 at positions 27 and 91. rAmphinase 2 is described in U.S. Pat No. 7,229,824. The amino acid sequence of rAmphinase 2 is provided in SEQ ID NO: 4.

In certain embodiments, the composition comprising a ribonuclease may use a personal lubricant as a vehicle to deliver and stabilise the ribonuclease. The term "personal lubricant" may be used interchangeably with the term "sexual lubricant" throughout this disclosure. The personal lubricant compositions comprising a ribonuclease can be in the form of a gel, liquid, cream, ointment, lotion, solution, suspension, or a spray.

A number of brands of personal lubricants are known, for example, K-Y jelly, Astroglide, Durex Play, Sylk, Elbow Grease, Good Clean Love, Gynol II, ID Glide Ultra, PRE, Replens, Slippery Stuff and Sliquid Organic, etc. The present disclosure provides personal lubricants that comprise a ribonuclease.

In one embodiment, the personal lubricant comprises ranpirnase or other ribonucleases and glycerol and/or a cellulose derivative. The cellulose derivative may include hydroxyethyl cellulose, sodium carboxymethyl cellulose and/or cellulose polymer.

In one embodiment, the personal lubricant comprises ranpirnase and one or more excipients selected from the group consisting of water, glycerin, propylene glycol, sorbitol, ethers or esters of cellulose such as hydroxyethylcellulose, dimethicone, cyclomethicone, dimethicone/vinyl dimethicone crosspolymer, vegetable oil, PEG/PPG-18/18 Dimethicone, Propanediol, Sodium Chloride, 1,2-Hexanediol, Dimethiconol, Caprylhydroxamic Acid, Caprylyl Glycol, lactic acid, chlorhexidine gluconate, gluconolactone, methylparaben, propyl paraben, benzoic acid, Polyquaternium 15, and sodium hydroxide.

In one embodiment, the personal lubricant comprises ranpirnase and a water-based personal lubricant comprising one or more excipients selected from the group consisting of water, glycerin, propylene glycol, sorbitol, ethers or esters of cellulose such as hydroxyethylcellulose, chlorhexidine gluconate, gluconolactone, parabens such as methylparaben and propylparaben, benzoic acid, Polyquaternium 15, and sodium hydroxide.

In another embodiment, the personal lubricant comprises ranpirnase and a silicone-based personal lubricant comprising one or more excipients selected from the group consisting of dimethicone, cyclomethicone, dimethicone/vinyl dimethicone crosspolymer, and vegetable oil including coconut oil, olive oil, etc.

In yet another embodiment, the personal lubricant comprises ranpirnase and a hybrid lubricant that combines excipients of a water-based lubricant and silicone-based ingredients. In one embodiment, such hybrid lubricant comprises Glycerin, Dimethicone, Purified Water, Cyclomethicone, PEG/PPG-18/18 Dimethicone, Propanediol, Sodium Chloride, 1,2-Hexanediol, Dimethiconol, Caprylhydroxamic Acid, and Natural Flavors.

In one embodiment, the personal lubricant comprises ranpirnase and pharmaceutically acceptable excipients selected from the group consisting of water, glycerin, ethers or esters of cellulose such as hydroxyethylcelluose, chlorhexidine gluconate, gluconolactone, methylparaben, and sodium hydroxide. In another embodiment, the personal lubricant comprises ranpirnase and pharmaceutically acceptable excipients selected from the group consisting of water, glycerin, propylene glycol, sorbitol, hydroxyethylcellulose, benzoic acid, methylparaben, and sodium hydroxide, as pharmaceutically acceptable excipients.

In yet another embodiment, the personal lubricant comprises ranpirnase and pharmaceutically acceptable excipients selected from the group consisting of Glycerin, Propylene Glycol, Polyquaternium 15, Methylparaben, and Propylparaben.

In yet another embodiment, the personal lubricant comprises ranpirnase and pharmaceutically acceptable excipients selected from the group consisting of dimethicone, cyclomethicone, dimethicone/vinyl dimethicone crosspolymer, and a vegetable oil such as coconut oil, olive oil, etc. In yet another embodiment, the personal lubricant comprises ranpirnase and pharmaceutically acceptable excipients selected from the group consisting of dimethicone and cyclomethicone.

In yet another embodiment, the personal lubricant comprises ranpirnase and pharmaceutically acceptable excipients selected from the group consisting of Glycerin, Dimethicone, Purified Water, Cyclomethicone, PEG/PPG-18/18 Dimethicone, Propanediol, Sodium Chloride, 1,2-Hexanediol, Dimethiconol, Caprylhydroxamic Acid, and Natural Flavors.

In yet another embodiment, the personal lubricant comprises ranpirnase and pharmaceutically acceptable excipients selected from the group consisting of Purified Water, Propylene Glycol, Hydroxyethylcellulose, Caprylyl Glycol, Caprylhydroxamic Acid, Propanediol, Polyquaternium 15, and Lactic Acid.

In some embodiments, the ribonuclease is present in the composition at a concentration of about 0.1 mg/mL to about 10 mg/mL, such as, about 0.1, 0.2, 0.3, 0.4, 0.5, 0.6, 0.7, 0.8, 0.9, 1.0, 2, 3, 4, 5, 6, 7, 8, 9, or 10 mg/mL, including values and ranges therebetween. Preferably and advantageously with respect to a combination of efficacy and viability, however, the concentration range is lower. Such lower concentrations are particularly preferred when the ribonuclease is ranpirnase. See Figs. 2A and 2B. Suitably low concentrations include where the ribonuclease is present in a range of about 2 µg/ml to about 200 µg/ml, preferably, about 5 µg/ml to about 100 µg/ml and most preferably about 5 µg/ml to about 50 µg/ml. As used herein, the term "about" represents a 10% variance of the indicated value, unless otherwise specified as +/-20%. Ranpirnases in these concentrations do not result in decreased host cell viability. Thus, in preferable aspects, the host cell viability, remains about 100%. See Fig.2B.

In some other embodiments, the ribonuclease is present in the composition at a concentration of about 0.1% to about 10% w/w, such as, about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, or 10% w/w, including values and ranges therebetween. For example, the ribonuclease may be present in the composition at a concentration of about 1% to about 10%, about 0.5% to about 5%, about 1% to about 5%, or about 5% to about 10% w/w, including values and ranges therebetween.

In yet some other embodiments, the ribonuclease is present in the composition at a concentration of about 0.1% to about 10% w/v, such as, about 0.1%, 0.2%, 0.3%, 0.4%, 0.5%, 0.6%, 0.7%, 0.8%, 0.9%, 1.0%, 2%, 3%, 4%, 5%, 6%, 7%, 8%, 9%, or 10% w/v, including values and ranges therebetween.

The present disclosure also provides a method for prophylactically treating a subject for sexually-transmitted viral infections comprises transfecting a gene encoding ranpirnase or other ribonucleases in a bacterium and administering the transfected bacterium to a subject For example, *E. coli* and *Lactobacillus* are part of normal microbial flora residing in mucosal tissues such as vagina and rectum. A gene or a DNA sequence encoding ranpirnase or other ribonucleases can be transfected into *E. coli* or *Lactobacillus* and the transfected/modified *E.coli* or *Lactobacillus* are introduced into the subject. The modified *E. coli* or *Lactobacillus* would enter the mucosal tissues of the subject and produce ranpirnase.

Alternatively, it is disclosed that other bacteria could be used to prophylactically protect other tissues against other viral infections. For example, probiotic *E. coli* comprising transfected ranpirnase DNA could be used to protect against viral infections of the digestive tract. Similarly, modified saprophytic Streptococci could be delivered to protect a subject's external genitalia against HIV.

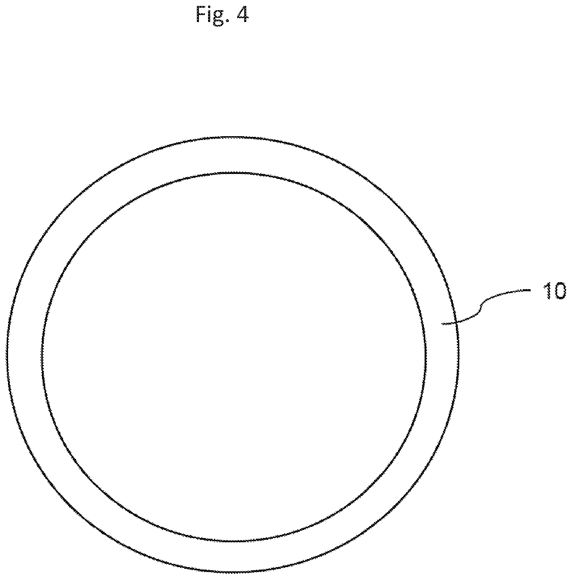

In certain other embodiments, the present disclosure provides an intravaginal ring 10 (Fig. 4) made of a suitably porous and biocompatible material mat is impregnated with ranpirnase other suitable ribonucleases. Intravaginal rings are known and are used to deliver birth control drugs as well as dapivirine (a candidate microbicide). The composition of such a ring is disclosed in Holt, et al., Antimicrobial Agents and Chemotherapy, Vol. 59, No. 7, pp. 3761 - 3770 (July, 2015).

When the intravaginal ring impregnated with ranpirnase is inserted into the vagina, the ranpirnase is released and would confer protection against viral infections. The ranpirnase may be mixed with other agents, e.g. dispersing agents, prior to applying to the intravaginal rings

The prophylactic treatment compositions of the present disclosure protect subjects from sexually-transmitted viral infections. In certain embodiments, the prophylactic treatment compositions protect subjects from infections caused by a virus selected from the group consisting of herpes simplex viruses (HSV), human papillomaviruses (HPV), human immunodeficiency virus (HIV), hepatitis B and C virus, and cytomegalovirus. Thus, in particular aspects, HIV infection in normal cells from rectal explants is inhibited after incubation with concentrations or ranpirnase ranging from 6 µg/ml to 66 µg/ml. Thus, in particular aspects, the compositions disclosed herein, while applied to the surface of the cell, allow penetration of ribonuclease (e.g., ranpirnase) into the cell where the HIV, or other viral RNA, is degraded, thus preventing the infection from establishing a foothold in the cell. The prophylactic treatment compositions of the present disclosure may protect subjects from acquiring sexually-transmitted diseases such as Acquired Immunodeficiency Syndrome (AIDS), anal warts, and genital warts.

The prophylactic treatment is intended to be administered topically, i.e. to a surface of the patient's body such as the skin or mucous membranes. Preferably, the composition is administered to the patient's skin, in particular, in body regions mat are exposed to sexually-transmitted viruses. The topical administration according to the present invention includes vaginal, extra-vaginal (outside of the vagina), intra-vaginal (inside the vagina), anal, peri-anal (surrounding the anus) and intra-anal (inside the anus) administration.

The composition may be applied topically prior to or during the exposure to sexually-transmitted viruses, e.g., prior to or during sexual intercourse.

The compositions of the present disclosure may be applied multiple times during the day. For example, in one embodiment, the prophylactic treatment comprises topically applying the ranpirnase compositions once, twice, three times, four times, five times, six times, seven times, eight times, nine times, or ten times in a day.

This disclosure is further illustrated by the following additional examples that should not be construed as limiting.

### EXAMPLES:

### Example 1

Three rectal tissue explants were obtained from six healthy volunteers (three from each volunteer). Fifteen explants were used to test the prophylactic effect of ranpirnase against HTV and three explants were used in an MTT assay to check whether ranpirnase caused cellular toxicity.

To test the prophylactic effect of ranpirnase against HIV, five solutions were prepared as follows:
1. 10⁵ TCID₅₀ of-HIV-1_{BaL} together with 0.06 µg/mL ranpirnase (test),
2. 10⁵ TCID₅₀ of-HIV-1_{BaL} together with 0.6 µg/mL ranpirnase (test),
3. 10⁵ TCID₅₀ of-HIV-1_{BaL} together with 6 µg/mL ranpirnase (test),
4. 10⁵ TCID₅₀ of-HIV-1_{BaL} together with 66 µg/mL ranpirnase (test), and
5. 10⁵ TCID₅₀ of-HIV-1_{BaL} with no ranpirnase (control).

The fifteen tissue explants were arranged into five groups of three. Each group of explants was incubated for two hours with one of the mixtures. The tissue explants were then washed multiple times and cultured at 37 °C and 5% CO₂ for fourteen days. Supernatants were collected on Days 3, 7, 10, and 14. The culture medium was prepared using equal parts of complete RMPI and Zosyn® 50 mg/mL. 500 mL of complete RMPI were prepared by mixing:
90% RPMI 1640 - 445 mL,
10% Fetal Bovine Serum - 50 mL, and
1% Antibiotic/Antimycotic 5 - 5.0 mL.

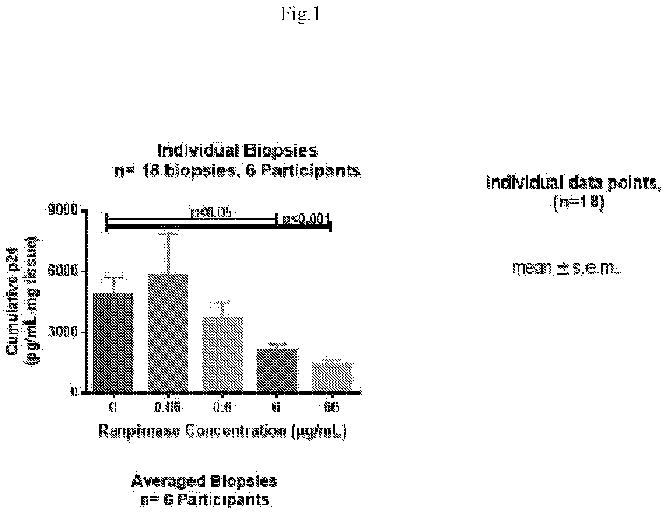

The severity of HIV infection of the tissue explants was determined by assaying the supernatant for HIV-1 p24 antigen using the AlphaLISA platform. The efficacy endpoint was the tissue explant weight adjusted Day 14 cumulative HIV-1 p24. As shown in Figures 1-3, increasing concentration of ranpirnase caused a dose dependent reduction in HIV-1 p24 antigen in the tissue explants. The statistical significance values (p values) are shown in Figures 1-3; whether the values were averaged or not, the results for 0.00 µg/mL, 0.06 µg/mL, and 0.6 µg/mL of ranpirnase were below the 5% level of significance. And, the results for 6.00 µg/mL and 66 µg/mL of ranpirnase were below the 1% level of significance when the results of each group of three tissue explants were averaged, and below the 0.1% level of significance when the results of each tissue explant is taken individually.

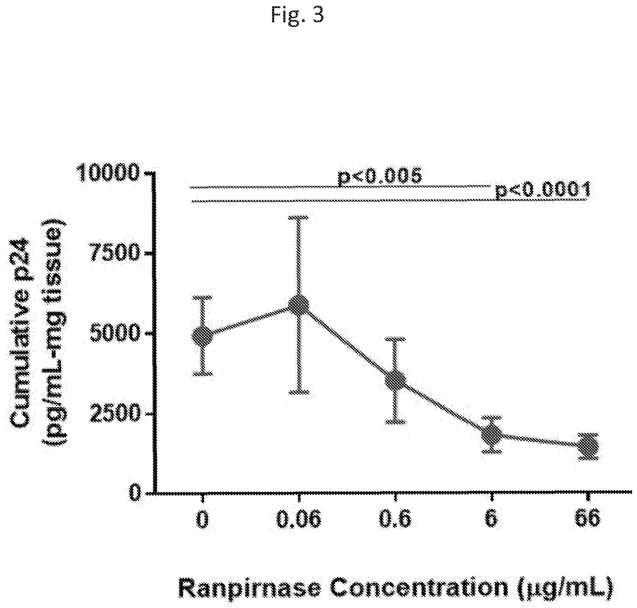

In each of Figures 1-3, the standard error of the mean is also shown by the vertical lines shown by themselves in Figure 3 and superposed on the bars in Figures 1 and 2.

Three tissue explants exposed to ranpirnase alone were tested for cell viability using an MTT assay. The results of the assay showed that ranpirnase did not induce cellular toxicity. See Fig.2B.

This experiment showed that tissue explants exposed to ranpirnase, in particular, to the ranpirnase concentrations of 6 µg/mL and greater, developed increased resistance to HIV infection, i.e. ranpirnase had a prophylactic effect.

## Claims

1. A composition comprising a ribonuclease and, optionally, one or more pharmaceutically acceptable excipients for use in prophylactically treating a subject from contracting a sexually-transmitted viral infection, wherein said composition is applied topically, and wherein the ribonuclease is a member of the ribonuclease A superfamily.

2. The composition for use of claim 1, wherein the ribonuclease is selected from a group consisting of: ranpirnase, the '805 variant, Amphinase 2, and rAmphinase 2.

3. The composition for use of claim 1 or 2, wherein the composition is a personal lubricant.

4. The composition for use of any one of claims 1 to 3, wherein the composition is a gel, cream, ointment, lotion, solution, suspension, or a spray.

5. The composition for use of any one of claims 1 to 4, wherein the composition comprises glycerin, hydroxyethylcellulose, chlorhexidine gluconate, gluconolactone, methylparaben, and sodium hydroxide, as pharmaceutically acceptable excipients.

6. The composition for use of any one of claims 1 to 4, wherein the composition comprises glycerin, propylene glycol, sorbitol, hydroxyethylcellulose, benzoic acid, methylparaben, and sodium hydroxide, as pharmaceutically acceptable excipients.

7. The composition for use of any one of claims 1 to 6, wherein the ribonuclease is present in an amount from about 0.01% by weight to about 10% by weight, based on the total weight of the composition, optionally from about 0.1% by weight to about 1% by weight, based on the total weight of the composition.

8. The composition for use of any one of claims 1 to 6, wherein the ribonuclease is present in an amount of about 1%, about 5%, or about 10%, by weight, based on the total weight of the composition.

9. The composition for use of any one of claims 1 to 8, wherein the composition is applied prior to or during sexual intercourse.

10. The composition for use of any one of claims 1 to 9, wherein the composition is applied one to five times a day.

11. The composition for use of any one of claims 1 to 10, wherein the composition is applied topically to body regions that are exposed to sexually-transmitted viruses.

12. The composition for use of any one of claims 1 to 11, wherein the sexually-transmitted viruses are selected from the group consisting of herpes simplex viruses (HSV), human papillomaviruses (HPV), human immunodeficiency virus (HIV), hepatitis B virus, hepatitis C virus, and cytomegalovirus.

13. The composition for use of any of claims 1 to 12, wherein the subject cell viability is about 100% following administration of the compound.

14. The composition for use of any of claims 1 to 13, wherein ranpirnase is present at a concentration of about 5 µg/ml to about 100 µg/ml or about 6 µg/ml to about 66 µg/ml.

## Patentansprüche

1. Zusammensetzung, umfassend eine Ribonuklease und wahlweise einen oder mehr pharmazeutisch annehmbare Hilfsstoffe zur Anwendung bei der prophylaktischen Behandlung eines Subjekts, an einer sexuell übertragbaren Virusinfektion zu erkranken, wobei die Zusammensetzung topisch angewendet wird, und wobei die Ribonuklease ein Mitglied der Ribonuklease A-Superfamilie ist.

2. Zusammensetzung zur Anwendung nach Anspruch 1, wobei die Ribonuklease aus einer Gruppe bestehend aus den folgenden ausgewählt ist: Ranpirnase, der '805-Variante, Amphinase 2 und rAmphinase 2.

3. Zusammensetzung zur Anwendung nach Anspruch 1 oder 2, wobei die Zusammensetzung ein persönliches Gleitmittel ist.

4. Zusammensetzung zur Anwendung nach einem der Ansprüche 1 bis 3, wobei die Zusammensetzung ein Gel, eine Creme, eine Salbe, eine Lotion, eine Lösung, eine Suspension oder ein Spray ist.

5. Zusammensetzung zur Anwendung nach einem der Ansprüche 1 bis 4, wobei die Zusammensetzung Glycerin, Hydroxyethylcellulose, Chlorhexidingluconat, Gluconolacton, Methylparaben und Natriumhydroxid als pharmazeutisch annehmbare Hilfsstoff umfasst.

6. Zusammensetzung zur Anwendung nach einem der Ansprüche 1 bis 4, wobei die Zusammensetzung Glycerin, Propylenglycol, Sorbitol, Hydroxyethylcellulose, Benzoesäure, Methylparaben und Natriumhydroxid als pharmazeutische annehmbare Hilfsstoffe umfasst.

7. Zusammensetzung zur Anwendung nach einem der Ansprüche 1 bis 6, wobei die Ribonuklease jeweils basierend auf dem Gesamtgewicht der Zusammensetzung in einer Menge von etwa 0,01 Gew% bis etwa 10 Gew% vorliegt, wahlweise jeweils basierend auf dem Gesamtgewicht der Zusammensetzung in einer Menge von etwa 0,1 Gew% bis etwa 1 Gew%.

8. Zusammensetzung zur Anwendung nach einem der Ansprüche 1 bis 6, wobei die Ribonuklease jeweils basierend auf dem Gesamtgewicht der Zusammensetzung in einer Menge von etwa 1 Gew%, etwa 5 Gew% oder etwa 10 Gew% vorliegt.

9. Zusammensetzung zur Anwendung nach einem der Ansprüche 1 bis 8, wobei die Zusammensetzung vor oder während eines Geschlechtsverkehrs angewendet wird.

10. Zusammensetzung zur Anwendung nach einem der Ansprüche 1 bis 9, wobei die Zusammensetzung ein- bis fünfmal täglich angewendet wird.

11. Zusammensetzung zur Anwendung nach einem der Ansprüche 1 bis 10, wobei die Zusammensetzung auf denjenigen Körperregionen, die sexuell übertragbaren Viren ausgesetzt sind, topisch angewendet wird.

12. Zusammensetzung zur Anwendung nach einem der Ansprüche 1 bis 11, wobei die sexuell übertragbaren Viren aus der Gruppe bestehend aus den Herpes-simplex-Viren (HSV), Humanen Papillomviren (HPV), dem Humanen Immunschwächevirus (HIV), Hepatitis-B-Virus, Hepatitis-C-Virus und Cytomegalie-Virus ausgewählt sind.

13. Zusammensetzung zur Anwendung nach einem der Ansprüche 1 bis 12, wobei die gegenständliche Zellviabilität nach der Verabreichung der Verbindung etwa 100 % ist.

14. Zusammensetzung zur Anwendung nach einem der Ansprüche 1 bis 13, wobei Ranpirnase in einer Konzentration von etwa 5 µg/ml bis etwa 100 µg/ml oder etwa 6 µg/ml bis etwa 66 µg/ml vorliegt.

## Revendications

1. Composition comprenant une ribonucléase et, éventuellement, un ou plusieurs excipients pharmaceutiquement acceptables à utiliser dans le traitement prophylactique d'un sujet contre la contraction d'une infection virale sexuellement transmissible, dans laquelle ladite composition est appliquée par voie topique, et dans laquelle la ribonucléase est un membre de la superfamille des ribonucléases A.

2. Composition à utiliser selon la revendication 1, dans laquelle la ribonucléase est choisie dans un groupe comprenant : la ranpirnase, le variant '805, l'amphinase 2 et la rAmphinase 2.

3. Composition à utiliser selon la revendication 1 ou 2, dans laquelle la composition est un lubrifiant personnel.

4. Composition à utiliser selon l'une quelconque des revendications 1 à 3, dans laquelle la composition est un gel, une crème, une pommade, une lotion, une solution, une suspension ou un spray.

5. Composition à utiliser selon l'une quelconque des revendications 1 à 4, dans laquelle la composition comprend de la glycérine, de l'hydroxyéthylcellulose, du gluconate de chlorhexidine, de la gluconolactone, du méthylparabène et de l'hydroxyde de sodium, comme excipients pharmaceutiquement acceptables.

6. Composition à utiliser selon l'une quelconque des revendications 1 à 4, dans laquelle la composition comprend de la glycérine, du propylène glycol, du sorbitol, de l'hydroxyéthylcellulose, de l'acide benzoïque, du méthylparabène et de l'hydroxyde de sodium, comme excipients pharmaceutiquement acceptables.

7. Composition à utiliser selon l'une quelconque des revendications 1 à 6, dans laquelle la ribonucléase est présente en une quantité d'environ 0,01% en poids à environ 10% en poids, par rapport au poids total de la composition, éventuellement d'environ 0,1% en poids à environ 1% en poids, par rapport au poids total de la composition.

8. Composition à utiliser selon l'une quelconque des revendications 1 à 6, dans laquelle la ribonucléase est présente en une quantité d'environ 1%, d'environ 5% ou d'environ 10%, en poids, par rapport au poids total de la composition.

9. Composition à utiliser selon l'une quelconque des revendications 1 à 8, dans laquelle la composition est appliquée avant ou pendant un rapport sexuel.

10. Composition à utiliser selon l'une quelconque des revendications 1 à 9, dans laquelle la composition est appliquée une à cinq fois par jour.

11. Composition à utiliser selon l'une quelconque des revendications 1 à 10, dans laquelle la composition est appliquée localement sur des régions corporelles qui sont exposées à des virus sexuellement transmissibles.

12. Composition à utiliser selon l'une quelconque des revendications 1 à 11, dans laquelle les virus sexuellement transmissibles sont choisis dans le groupe constitué par les virus de l'herpès simplex (VHS), les virus du papillome humain (VPH), le virus de l'immunodéficience humaine (VIH), le virus de l'hépatite B, le virus de l'hépatite C et le cytomégalovirus.

13. Composition à utiliser selon l'une quelconque des revendications 1 à 12, dans laquelle la viabilité cellulaire du sujet est d'environ 100% après l'administration du composé.

14. Composition à utiliser selon l'une quelconque des revendications 1 à 13, dans laquelle la ranpirnase est présente à une concentration d'environ 5 µg/ml à environ 100 µg/ml ou d'environ 6 µg/ml à environ 66 µg/ml.


### SEQUENCE LISTING

<110> Tamir Biotechnology, Inc. Squiquera, Luis
<120> PROPHYLACTIC PROTECTION AGAINST VIRAL INFECTIONS
<130> TAMI-016/01WO
<150> US 62/367,050
   <151> 2016-07-26
<160> 4
<170> PatentIn version 3.5
<210> 1
   <211> 104
   <212> PRT
   <213> Rana pipiens
<400> 1
   Glu Asp Trp Leu Thr Phe Gln Lys Lys His Ile Thr Asn Thr Arg Asp
   1               5                   10                  15
   Val Asp Cys Asp Asn Ile Met Ser Thr Asn Leu Phe His Cys Lys Asp
               20                  25                  30
   Lys Asn Thr Phe Ile Tyr Ser Ala Pro Glu Pro Val Lys Ala Ile Cys
           35                  40                  45
   Lys Gly Ile Ile Ala Ser Lys Asn Val Leu Thr Thr Ser Glu Phe Tyr
       50                  55                  60
   Leu Ser Asp Cys Asn Val Thr Ser Arg Pro Cys Lys Tyr Lys Leu Lys
   65                  70                  75                  80
   Lys Ser Thr Asn Lys Phe Cys Val Thr Cys Glu Asn Gln Ala Pro Val
                   85                  90                  95
   His Phe Val Gly Val Gly Ser Cys
               100
<210> 2
   <211> 104
   <212> PRT
   <213> Rana pipiens
<400> 2
   Glu Asp Trp Leu Thr Phe Gln Lys Lys His Val Thr Asn Thr Arg Asp
   1               5                   10                  15
   Val Asp Cys Asn Asn Ile Met Ser Thr Asn Leu Phe His Cys Lys Asp
                   20                  25                  30
   Lys Asn Thr Phe Ile Tyr Ser Arg Pro Glu Pro Val Lys Ala Ile Cys
           35                  40                  45
   Lys Gly Ile Ile Ala Ser Lys Asn Val Leu Thr Thr Ser Glu Phe Tyr
       50                  55                  60
   Leu Ser Asp Cys Asn Val Thr Ser Arg Pro Cys Lys Tyr Lys Leu Lys
   65                  70                  75                  80
   Lys Ser Thr Asn Lys Phe Cys Val Thr Cys Glu Asn Gln Ala Pro Val
                   85                  90                  95
   His Phe Val Gly Val Gly Arg Cys
               100
<210> 3
   <211> 114
   <212> PRT
   <213> Rana pipiens
<400> 3
   Lys Pro Lys Glu Asp Arg Glu Trp Glu Lys Phe Lys Thr Lys His Ile
   1               5                   10                  15
   Thr Ser Gln Ser Val Ala Asp Phe Asn Cys Asn Arg Thr Met Asn Asp
               20                  25                  30
   Pro Ala Tyr Thr Pro Asp Gly Gln Cys Lys Pro Ile Asn Thr Phe Ile
           35                  40                  45
   His Ser Thr Thr Gly Pro Val Lys Glu Ile Cys Arg Arg Ala Thr Gly
       50                  55                  60
   Arg Val Asn Lys Ser Ser Thr Gln Gln Phe Thr Leu Thr Thr Cys Lys
   65                  70                  75                  80
   Asn Pro Ile Arg Cys Lys Tyr Ser Gln Ser Asn Thr Thr Asn Phe Ile
                   85                  90                  95
   Cys Ile Thr Cys Arg Asp Asn Tyr Pro Val His Phe Val Lys Thr Gly
               100                 105                 110
   Lys Cys
<210> 4
   <211> 115
   <212> PRT
   <213> Rana pipiens
<400> 4
   Met Lys Pro Lys Glu Asp Arg Glu Trp Glu Lys Phe Lys Thr Lys His
   1               5                   10                  15
   Ile Thr Ser Gln Ser Val Ala Asp Phe Asn Cys Asn Arg Thr Met Asn
               20                  25                  30
   Asp Pro Ala Tyr Thr Pro Asp Gly Gln Cys Lys Pro Ile Asn Thr Phe
           35                  40                  45
   Ile His Ser Thr Thr Gly Pro Val Lys Glu Ile Cys Arg Arg Ala Thr
       50                  55                  60
   Gly Arg Val Asn Lys Ser Ser Thr Gln Gln Phe Thr Leu Thr Thr Cys
   65                  70                  75                  80
   Lys Asn Pro Ile Arg Cys Lys Tyr Ser Gln Ser Asn Thr Thr Asn Phe
                   85                  90                  95
   Ile Cys Ile Thr Cys Arg Asp Asn Tyr Pro Val His Phe Val Lys Thr
               100                 105                 110
   Gly Lys Cys
           115